# EUROPÄISCHE PATENTSCHRIFT

(11) **EP 2 720 709 B1**
(45) Veröffentlichungstag und Bekanntmachung des Hinweises auf die Patenterteilung: **09.08.2017**
(21) Anmeldenummer: 12727648.3
(22) Anmeldetag: 13.06.2012
(51) Int. Cl.: A61K 38/00, C07K 14/48, C07K 14/475

(54) **CILIARY-NEUROTROPHIC-FACTOR-VARIANTEN**
CILIARY NEUROTROPHIC FACTOR VARIANTS
VARIANTS DU FACTEUR NEUROTROPHIQUE CILIAIRE

(30) Priorität: 18.06.2011 DE 102011104822
(43) Veröffentlichungstag der Anmeldung: 23.04.2014
(73) Patentinhaber: Christian-Albrechts-Universität zu Kiel, 24118 Kiel (DE)
(72) Erfinder: GRÖTZINGER, Joachim, 24214 Altwittenbek (DE); ROSE-JOHN, Stefan, 24211 Schellhorn (DE); SCHELLER, Jürgen, 41469 Neuss (DE); AURICH, Matthias, 25899 Niebüll (DE)
(74) Vertreter: Uexküll & Stolberg
(86) Internationale Anmeldenummer: PCT/EP2012/061143
(87) Internationale Veröffentlichungsnummer: WO 2012/175378

(56) Entgegenhaltungen:
- WO-A2-95/00852
- WO-A2-2005/014641
- MARCO DI A ET AL: "AGONISTIC AND ANTAGONISTIC VARIANTS OF CILIARY NEUROTROPHIC FACTOR (CNTF) REVEAL FUNCTIONAL DIFFERENCES BETWEEN MEMBRANE-BOUND AND SOLUBLE CNTF ALPHA-RECEPTOR", JOURNAL OF BIOLOGICAL CHEMISTRY, AMERICAN SOCIETY FOR BIOCHEMISTRY AND MOLECULAR BIOLOGY, US, Bd. 272, Nr. 37, 12. September 1997 (1997-09-12), Seiten 23069-23075, XP002069814, ISSN: 0021-9258, DOI: 10.1074/JBC.272.37.23069
- SAGGIO I ET AL: "CNTF VARIANTS WITH INCREASED BIOLOGICAL POTENCY AND RECEPTOR SELECTIVITY DEFINE A FUNCTIONAL SITE OF RECEPTOR INTERACTION", EMBO JOURNAL, OXFORD UNIVERSITY PRESS, SURREY, GB, Bd. 14, Nr. 13, 1. Januar 1995 (1995-01-01), Seiten 3045-3054, XP002056939, ISSN: 0261-4189
- PANAYOTATOS N ET AL: "EXCHANGE OF A SINGLE AMINO ACID INTERCONVERTS THE SPECIFIC ACTIVITY AND GEL MOBILITY OF HUMAN AND RAT CILIARY NEUROTROPHIC FACTORS", JOURNAL OF BIOLOGICAL CHEMISTRY, AMERICAN SOCIETY FOR BIOCHEMISTRY AND MOLECULAR BIOLOGY, US, Bd. 268, Nr. 25, 1. Januar 1993 (1993-01-01), Seiten 19000-19003, XP000941479, ISSN: 0021-9258
- B. SCHUSTER: "Signaling of Human Ciliary Neurotrophic Factor (CNTF) Revisited. THE INTERLEUKIN-6 RECEPTOR CAN SERVE AS AN alpha -RECEPTOR FOR CNTF", JOURNAL OF BIOLOGICAL CHEMISTRY, Bd. 278, Nr. 11, 7. März 2003 (2003-03-07) , Seiten 9528-9535, XP55037440, ISSN: 0021-9258, DOI: 10.1074/jbc.M210044200

## Beschreibung

Die Erfindung betrifft Ciliary-Neurotrophic-Factor (CNTF)-Varianten, welche wegen des Verlustes der Affinität für den Interleukin-6 Rezeptor (IL-6R) besonders nebenwirkungsarm als Therapeutikum bei mit in CNTF in Zusammenhang stehenden Störungen oder Erkrankungen eingesetzt werden können.

Bei der Entwicklung neuer Medikamente, die nicht nur sicher und effektiv das Gewicht reduzieren, sondern gleichzeitig die häufig mit der Adipositas vergesellschafteten metabolischen Entgleisungen wie Insulinresistenz und Glucoseintoleranz positiv beeinflussen können, ist eine Klasse körpereigener Moleküle zum Gegenstand aktueller Untersuchungen geworden. Es handelt sich dabei um so genannte Glykoprotein130 (gp130)-Liganden aus der Gruppe der Interleukin(IL)-6 artigen Zytokine, wobei insbesondere der Ciliary Neurotrophic Factor (CNTF) und seine Analoga als potentielle Antiadiposita erforscht werden (Febbraio MA. gp130 receptor ligands as potential therapeutic targets for obesity. J Clin Invest 2007; 117(4):841-9).

CNTF wurde ursprünglich als trophischer Faktor, der das Überleben von Neuronen im Ziliarganglion von Hühnerembryonen fördert, identifiziert (Adler R, Landa KB, Manthorpe M, Varon S. Cholinergic neuronotrophic factors: intraocular distribution of trophic activity for ciliary neurons. Science 1979; 204(4400):1434-6). Weitere Untersuchungen ergaben, dass dieser Effekt nicht nur bei parasympathischen, sondern darüber hinaus bei anderen peripheren wie sympathischen, sensorischen und motorischen (Barbin G, Manthorpe M, Varon S. Purification of the chick eye ciliary neuronotrophic factor. J Neurochem 1984; 43(5):1468-78; Sendtner M, Kreutzberg GW, Thoenen H. Ciliary neurotrophic factor prevents the degeneration of motor neurons after axotomy. Nature 1990; 345(6274):440-1) aber auch verschiedenen zentralen Nervenzellen nachweisbar war (Ip NY, Li YP, van dS, I, Panayotatos N, Alderson RF, Lindsay RM. Ciliary neurotrophic factor enhances neuronal survival in embryonic rat hippocampal cultures. J Neurosci 1991; 11(10):3124-34; Hagg T, Quon D, Higaki J, Varon S. Ciliary neurotrophic factor prevents neuronal degeneration and promotes low affinity NGF receptor expression in the adult rat CNS. Neuron 1992; 8(1):145-58; Ip NY, McClain J, Barrezueta NX, Aldrich TH, Pan L, Li Y, Wiegand SJ, Friedman B, Davis S, Yancopoulos GD. The alpha component of the CNTF receptor is required for signaling and defines potential CNTF targets in the adult and during development. Neuron 1993; 10(1):89-102; Clatterbuck RE, Price DL, Koliatsos VE. Ciliary neurotrophic factor prevents retrograde neuronal death in the adult central nervous system. Proc Natl Acad Sci U S A 1993; 90(6):2222-6). CNTF ist ein evolutionär stark konserviertes Molekül, dessen physiologische Funktionen sich hauptsächlich auf die neuromuskuläre Achse beschränken (Perret D, Guillet C, Elson G, Froger J, Plun-Favreau H, Rousseau F, Chabbert M, Gauchert JM, Gascan H. Two different contact sites are recruited by cardiotrophinlike cytokine (CLC) to generate the CLC/CLF and CLC/sCNTFRalpha composite cytokines. J Biol Chem 2004; 279(42):43961-70). Strukturell und funktionell weist CNTF einige Besonderheiten auf, durch welche sich dieser Faktor von den meisten anderen Zytokinen der gp130-Gruppe unterscheidet. Zum einen besitzt er kein Signalpeptid, kann somit also auch nicht sezerniert werden. Dies führte schon frühzeitig zu der Vermutung, dass es sich hierbei um ein zytosolisch gelagertes und nur bei Schädigungen der Nervenfaser freigesetztes Protein handelt (Masiakowski P, Liu HX, Radziejewski C, Lottspeich F, Oberthuer W, Wong V, Lindsay RM, Furth ME, Panayotatos N. Recombinant human and rat ciliary neurotrophic factors. J Neurochem 1991; 57(3):1003-12.; Stöckli KA, Lottspeich F, Sendtner M, Masiakowski P, Carroll P, Gotz R, Lindholm D, Thoenen H. Molecular cloning, expression and regional distribution of rat ciliary neurotrophic factor. Nature 1989; 342(6252):920-3). Zum anderen ist CNTF bei hoher Konzentration dazu in der Lage, neben seinem originären α-Rezeptor (CNTFR), auch an den IL-6 Rezeptor (IL-6R) zu binden (Schuster B, Kovaleva M, Sun Y, Regenhard P, Matthews V, Grotzinger J, Rose-John S, Kallen KJ. Signaling of human ciliary neurotrophic factor (CNTF) revisited. The interleukin-6 receptor can serve as an alpha-receptor for CTNF. J Biol Chem 2003; 278(11):9528-35).

Aufgrund seines neurotrophen Potentials wurde CNTF als mögliches Therapeutikum bei verschiedenen neurodegenerativen Erkrankungen getestet. Tiermodelle konnten protektive Effekte (Anderson KD, Panayotatos N, Corcoran TL, Lindsay RM, Wiegand SJ. Ciliary neurotrophic factor protects striatal output neurons in an animal model of Huntington disease. Proc Natl Acad Sci U S A 1996; 93(14):7346-51.; Emerich DF, Winn SR, Hantraye PM, Peschanski M, Chen EY, Chu Y, McDermott P, Baetke EE, Kordower JH. Protective effect of encapsulated cells producing neurotrophic factor CNTF in a monkey model of Huntington's disease. Nature 1997; 386(6623):395-9) und eine Phase-I-Studie die Sicherheit von intrazerebral appliziertem CNTF bei Chorea Huntington nachweisen (Bloch J, Bachoud-Levi AC, Deglon N, Lefaucheur JP, Winkel L, Palfi S, Nguyen JP, Bourdet C, Gaura V, Remy P, Brugieres P, Boisse MF, Baudic S, Cesaro P, Hantraye P, Aebischer P, Peschanski M. Neuroprotective gene therapy for Huntington's disease, using polymer-encapsulated cells engineered to secrete human ciliary neurotrophic factor: results of a phase I study. Hum Gene Ther 2004; 15(10):968-75). Bei Patienten mit amyotropher Lateralsklerose hingegen zeigte das im Rahmen einer klinischen Phase-III-Studie subkutan injizierte CNTF keinen Einfluss auf die Progression der Krankheit (Miller RG, Petajan JH, Bryan WW, Armon C, Barohn RJ, Goodpasture JC, Hoagland RJ, Parry GJ, Ross MA, Stromatt SC. A placebo-controlled trial of recombinant human ciliary neurotrophic (rhCNTF) factor in amyotrophic lateral sclerosis. rhCNTF ALS Study Group. Ann Neurol 1996; 39(2):256-60). Dafür entwickelte sich bei einigen Patienten in dieser Studie ein dosisabhängiger Gewichtsverlust, der die schon zuvor beobachtete Kachexie nach systemischer Gabe von CNTF an Mäuse bestätigte (Henderson JT, Seniuk NA, Richardson PM, Gauldie J, Roder JC. Systemic administration of ciliary neurotrophic factor induces cachexia in rodents. J Clin Invest 1994; 93(6):2632-8). Infolgedessen konzentrierte man sich auf die Erschließung der Mechanismen, die zu einer solchen Reduktion des Körpergewichts führen, um sie anschließend gezielt beeinflussen zu können. Dabei bleibt aber festzustellen, dass CNTF kein physiologischer Gewichtsregulator ist und seine kachektischen Effekte ausschließlich nach exogener Verabreichung im Rahmen von Tierexperimenten und klinischen Studien beobachtet wurden.

Schon frühzeitig war bekannt, dass CNTF aus Ratten eine vierfach höhere Aktivität aufweist als menschliches CNTF (Masiakowski P, Liu HX, Radziejewski C, Lottspeich F, Oberthuer W, Wong V, Lindsay RM, Furth ME, Panayotatos N. Recombinant human and rat ciliary neurotrophic factors. J Neurochem 1991; 57(3):1003-12). Verantwortlich dafür ist eine Aminosäure an Position 63 des Proteins bei der es sich in der Ratte um Arginin (R) und beim Menschen um Glutamin (Q) handelt. Durch die Substitution Q63R konnte eine menschliche CNTF-Variante erzeugt werden, deren Aktivität der von Ratten-CNTF entspricht (Panayotatos N, Radziejewska E, Acheson A, Pearsall D, Thadani A, Wong V. Exchange of a single amino acid interconverts the specific activity and gel mobility of human and rat ciliary neurotrophic factors. J Biol Chem 1993; 268(25):19000-3). Weitere Untersuchungen zeigten, dass in gewissem Maße auch N- oder C-terminale Deletionen zu einer Aktivitätssteigerung führten oder die CNTF-Aktivität zumindest nicht negativ beeinflussten (Negro A, Corsa V, Corona G, Grandi C, Skaper SD, Callegaro L. Structure-function studies of human ciliary neurotrophic factor. Neurochem Res 1994; 19(2):223-7; Krüttgen A, Grotzinger J, Kurapkat G, Weis J, Simon R, Thier M, Schroder M, Heinrich P, Wollmer A, Comeau M. Human ciliary neurotrophic factor: a structurefunction analysis. Biochem J 1995; 309 (Pt 1):215-20). Diese Erkenntnisse führten schließlich zur Generierung von Axokine^{®}, einem CNTF-Analogon, das sich vom natürlichen humanen CNTF durch gezielte Veränderungen unterscheidet (Deletion der letzten 15 C-terminalen Aminosäuren, Austausch des Cysteins an Position 17 durch Alanin sowie des Glutamins an Position 63 durch Arginin) (Peterson WM, Wang Q, Tzekova R, Wiegand SJ. Ciliary neurotrophic factor and stress stimuli activate the Jak-STAT pathway in retinal neurons and glia. J Neurosci 2000; 20(11):4081-90) und das als potentielles Therapeutikum gegen Adipositas getestet wird (Preti A. Axokine (Regeneron). IDrugs 2003; 6(7):696-701). Vor kurzem wurde außerdem der Nachweis erbracht, dass die Aktivität von Axokine^{®} sogar bei gleichzeitiger Entfernung N- und C-terminaler Aminosäuren erhalten bleibt (Breusing K. Generierung und Charakterisierung eines verbesserten Ciliary Neurotrophic Factors (CNTF). Inauguraldissertation zur Erlangung der Doktorwürde der Medizinischen Fakultät der Christian-Albrechts-Universität zu Kiel 2007).

Das von der Firma Regeneron entwickeltes CNTF-Analogon Axokine^{®} wurde bereits als potentielles Therapeutikum gegen Adipositas getestet. Allerdings wurde bei klinischen Studien die Bildung von neutralisierenden Antikörpern gegen CNTF bei zahlreichen Patienten beobachtet, und diese Substanz kam nicht auf den Markt.

Die therapeutische Verwendung von CNTF bzw. CNTF-Analoga erwies sich bisher als sehr problematisch aufgrund der starken Nebenwirkungen, die mit ihrer systemischen Verabreichung verbunden sind. Die Tatsache, dass CNTF bei hoher Konzentration dazu in der Lage ist, neben seinem originären Rezeptor auch an den IL-6R zu binden, kann möglicherweise einige der IL-6R-vermittelten Begleiterscheinungen von systemisch appliziertem CNTF wie Fieber (Shapiro L, Zhang XX, Rupp RG, Wolff SM, Dinarello CA. Ciliary neurotrophic factor is an endogenous pyrogen. Proc Natl Acad Sci U S A 1993; 90(18):8614-8) oder die Induktion einer Akut-Phase-Antwort in der Leber (Dittrich F, Thoenen H, Sendtner M. Ciliary neurotrophic factor: pharmacokinetics and acute-phase response in rat. Ann Neurol 1994; 35(2):151-63) erklären. Diese Effekte könnten durch eine Monospezifität für den CNTFR vermieden werden.

Die bei klinischen Studien beobachtete Bildung von neutralisierenden Antikörpern gegen CNTF ist ein weiterer bisher sehr problematischer Aspekt des Einsatzes von CNTF als Medikament. Diese Antikörper können sowohl die therapeutische Wirkung des Medikamentes gefährden als auch mit dem körpereigenen CNTF interferieren, möglicherweise mit gravierenden Folgen. Daher haben sich nicht nur die Firma Regeneron, sondern auch die Firmen Xencor und Merck mit der Abschwächung der Immunogenizität von CNTF auseinandergesetzt (s. WO 02/070698 A2; US 2005/0064555 A1 und US 2005/0069987 A1).

CNTF besitzt wegen der Aktivierung des IL-6R eine B-Zell- und somit eine immunstimulierende Aktivität. Es ist also möglich, dass die beobachtete ausgeprägte Antikörperbildung auf die B-Zell-Stimulierung durch die Fähigkeit des verabreichten CNTF, den IL-6R zu binden und zu aktivieren, zu erklären ist. Eine CNTF-Variante, die nicht in der Lage ist, den IL-6R zu aktivieren, würde diese Eigenschaft möglicherweise nicht mehr besitzen. Da B-Zellen andererseits keinen CNTFR exprimieren, sollte eine solche CNTF-Variante nicht in der Lage sein, B-Zellen zu stimulieren. Es liegt somit nahe, dass damit die Antikörperbildung gegen die CNTF-Variante unterbleibt.

Es besteht dringender Bedarf an neuen Therapeutika gegen Adipositas und gegen neurologische Erkrankungen. CNTF-Varianten ohne oder mit geringer Affinität zum IL-6R, die es ermöglichen, IL-6R-bezogene Nebenwirkungen zu vermeiden, sind interessante Kandidaten für eine neue Generation von CNTF-Medikamenten gegen neurologische Erkrankungen und zur Regulation des Körpergewichts.

Der vorliegenden Erfindung liegt damit die Aufgabe zugrunde, neue CNTF-Varianten ohne oder mit geringer Affinität zum IL-6R bereitzustellen, die als Therapeutika ohne IL-6R-bezogene Nebenwirkungen verwendet werden können.

Erfindungsgemäß wird diese Aufgabe durch CNTF-Varianten mit den Merkmalen des Anspruch 1 gelöst. Die Unteransprüche geben vorteilhafte Ausführungen der Erfindung wieder.

Bei erfindungsgemäßen CNTF-Varianten handelt es sich um CNTF-Moleküle ohne oder mit geringer Affinität zum IL-6R, so dass diese Moleküle mit geringer Affinität als der CNTF an den IL-6R binden. Dieser Affinitätsverlust wird überraschenderweise durch eine einzige A-minosäurensubstitution, nämlich den Austausch vom Arginin-Rest an Position 28 vom humanen Wildtyp-CNTF (SEQ ID:NO 7) gegen einen nicht-positiv-geladenen Aminosäurerest, erreicht. Somit könnten IL-6R-vermittelte Begleiterscheinungen von systemisch appliziertem CNTF wie Fieber oder die Induktion einer Akut-Phase-Antwort in der Leber durch den von der Erfindung erreichten Affmitätsverlust für den IL-6R vermieden werden. Die erfindungsgemäßen CNTF-Varianten sollten außerdem nicht in der Lage sein, B-Zellen zu stimulieren, so dass die Antikörperbildung gegen diese CNTF-Moleküle möglicherweise unterbleiben könnte. Wegen dieser Möglichkeiten, IL-6R-bezogene Nebenwirkungen zu vermeiden, sind die erfindungsgemäßen CNTF-Varianten besonders geeignet als CNTF-Therapeutika.

Die Erfindung wird in den Figuren näher veranschaulicht. Es zeigen:
- Fig. 1: die Struktur von mutCNTF und NNTF-1;
- Fig. 2: das Alignment der Aminosäuresequenzen des humanen CNTF, mutCNTF und NNTF-1;
- Fig. 3: die PCR-Strategie zur Erzeugung von NNTF-1;
- Fig. 4: einen Proliferationsassay mit BAF/3 CNTFR-gp130-LIFR Zellen;
- Fig. 5: einen Proliferationsassay mit BAF/3 IL-6R-gp130-LIFR Zellen; und
- Fig. 6: einen Aktivitätsvergleich zwischen mutCNTF und NNTF-1.

Gegenstand der vorliegenden Erfindung sind CNTF-Varianten, welche den CNTFR als α-Rezeptor rekrutieren aber im, Gegensatz zum Wildtyp-CNTF, nicht oder nur in sehr geringem Maße mit dem IL-6R interagieren. Dies wird durch den erfindungsgemäßen Austausch vom Arginin-Rest an Position 28 vom humanen Wildtyp-CNTF (SEQ ID:NO 7) gegen einen nicht-positiv-geladenen Aminosäurerest erreicht.

Erfindungsgemäß handelt es sich bei dem nicht-positiv-geladenen Aminosäurerest bevorzugt um einen Alanin-, Asparagin-, Asparaginsäure-, Cystein-, Glutamin-, Glutaminsäure-, Glycin-, Isoleucin-, Leucin-, Methionin-, Phenylalanin-, Prolin-, Selenocystein-, Serin-, Threonin-, Tryptophan-, Tyrosin- oder Valin-Rest. Erfindungsgemäß erfolgt der Austausch an Position 28 vom humanen Wildtyp-CNTF (SEQ ID:NO 7) nicht durch folgende positiv-geladene A-minosäurereste: Histidin- oder Lysin-Rest.

In einer speziellen Ausführungsform der Erfindung erfolgt die erfindungsgemäße Aminosäurensubstitution bei der CNTF-Variante mutCNTF (SEQ ID:NO 3 und 4). SEQ ID:NO 8 stellt die allgemeine erfindungsgemäße Sequenz der CNTF-Varianten ausgehend von mutCNTF dar, bei denen an Position 15 der Austausch des Arginin-Restes gegen einen nicht-positiv-geladenen Aminosäurerest erfolgt. In einer bevorzugten Ausführung der Erfindung handelt es sich dabei um den Austausch von Arginin (R) an Position 28 vom humanen Wildtyp-CNTF (Position 15 von mutCNTF) gegen Glutaminsäure (E) (NNTF-1; SEQ ID:NO 5 und SEQ ID:NO 6).

Die Erfinder haben die CNTF-Variante mutCNTF (SEQ ID:NO 3 und 4) und die erfindungsgemäße R28E-CNTF-Variante (NNTF-1; SEQ ID:NO 5 und 6) wie in den Beispielen beschrieben zunächst in Form von Einschlusskörpern durch ein bakterielles System exprimiert, dann gereinigt und anschließend rückgefaltet. Darauf hin erfolgte die Aktivitätstestung auf zwei verschiedenen BAF/3-Zelllinien, die, neben den erforderlichen β-Untereinheiten, entweder den CNTFR oder den IL-6R exprimieren, wodurch der Spezifitätsnachweis für die CNTF-Variante erbracht wurde. Der Affinitätsverlust wird dadurch verdeutlicht, dass nur die Interaktion eines Interleukins mit dem entsprechenden Rezeptorsetting die Zellproliferation fördern kann.

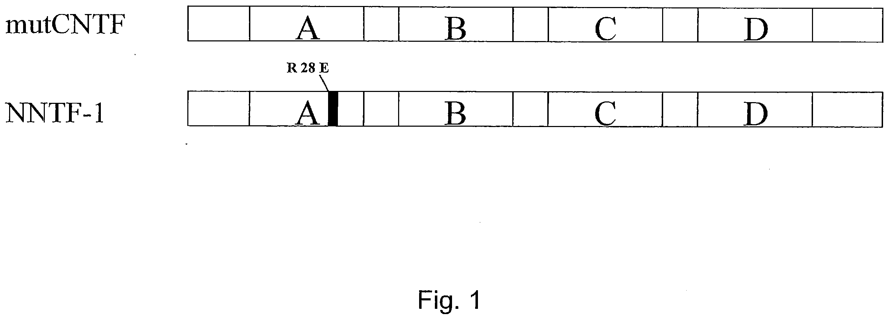

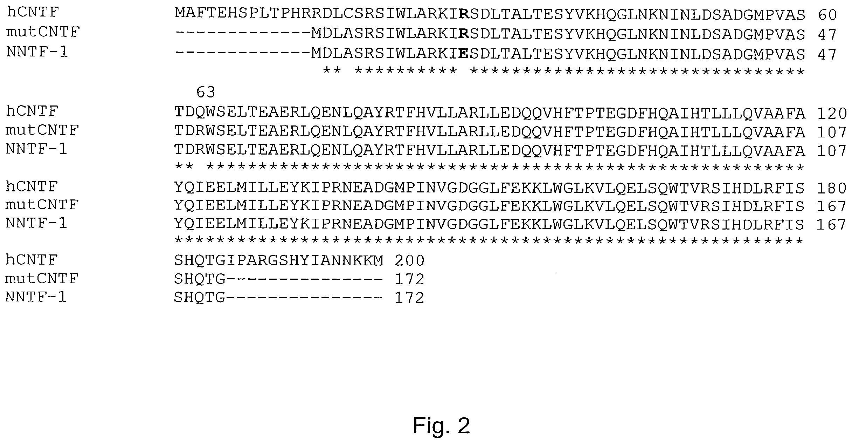

Fig. 1 zeigt die Proteine mutCNTF und NNTF-1 in Form sogenannter Bandmodelle, wobei die vier mit A bis D beschrifteten α-Helices als Strukturmerkmal der IL-6-Familie schematisch dargestellt sind. Bei der verwendeten CNTF-Variante mutCNTF handelt es sich um eine modifizierte Variante des CNTF-Derivats Axokine^{®} (C17A, Q63R, C-terminale Deletion von 15 Aminosäuren). Axokine^{®} weist eine 3- bis 5-fach höhere Wirksamkeit als das Wildtyp-CNTF (Preti A. Axokine (Regeneron). IDrugs 2003; 6(7):696-701) und wurde daher als Grundlage für diese Studien gewählt. In mutCNTF wurde zusätzlich der N-Terminus um 14 Aminosäuren verkürzt. Die Deletion der 14 N-terminalen Aminosäuren sollte keine Auswirkungen auf die Proteinfunktion haben, da sie kein Bestandteil der α-Helix A sind. In der erfmdungsgemäßen CNTF-Variante NNTF-1 wurde eine weitere Punktmutation eingefügt, welche für einen Aminosäureaustausch an der Stelle 28 (R28E, Nummerierung bezieht sich auf den Wildtyp-CNTF; SEQ ID:NO 7) codiert (Fig. 2).

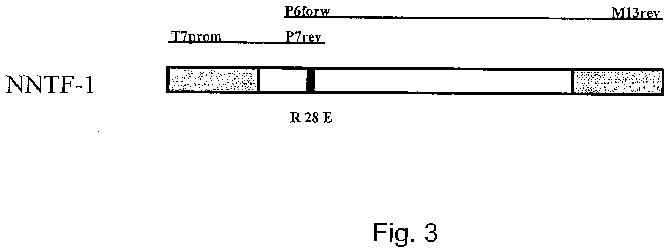

Als Template zur Erzeugung der NNTF-1 cDNA fungierte die cDNA des mutCNTF (SEQ ID:NO 3) im Vektor pPCR-Script-mutCNTF. Unter Verwendung der Primerpaare
T7prom [5 -GTAATACGACTCACTATAGGGC-3']
+ P6forw [5'-CAAGGAAGATTGAGTCAGACCTGACTG-3'] und
P7rev [5'-CAGTCAGGTCTGACTCAATCTTCCTTG-3']
+ M13rev: [5'-CAGGAAACAGCTATGACCAT-3']
wurden in zwei PCR-Reaktionen die beiden 5'-und 3'-NNTF-1 cDNA-Fragmente aus der cDNA von CNTF hergestellt und diese mittels SOE (Splicing by Overlapping Extension)-PCR (T7prom + M13rev) miteinander zu einer NNTF-1 cDNA verbunden (Fig. 3; Beispiel 1).

Die beiden C-terminal c-myc und His-getaggten rekombinanten Proteine mutCNTF und NNTF-1 wurden wie in Beispiel 2 beschrieben hergestellt.

Die Aktivitätstestung der Proteine mutCNTF und NNTF-1 erfolgte auf den murinen pro-B-Zelllinien BAF/3 CNTFR-gp130-LIFR bzw. BAF/3 IL-6R-gp130-LIFR (Beispiel 3). Sie exprimieren dank stabiler Transfektion neben den β-Rezeptoren gp130 und LIFR, als α-Rezeptor entweder den CNTFR oder den IL-6R, sodass nur ein zur Interaktion mit dem entsprechenden Rezeptorsetting befähigtes Interleukin ihre Proliferation fördern kann.

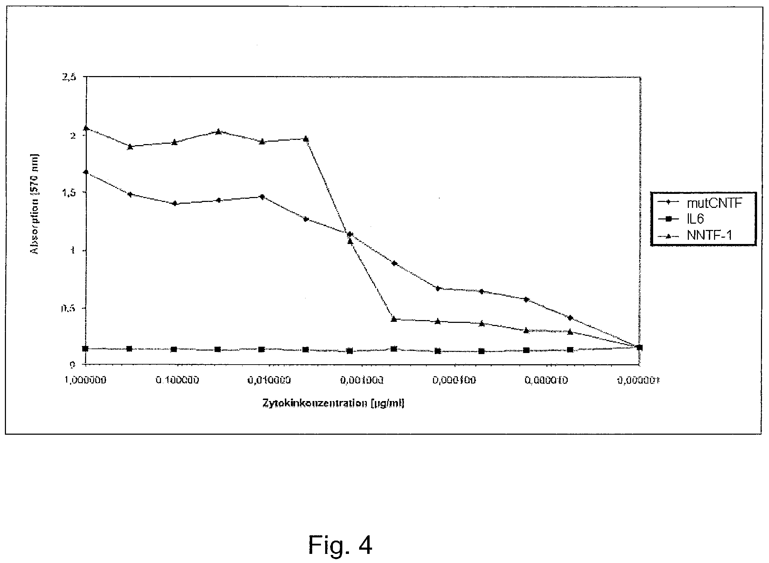

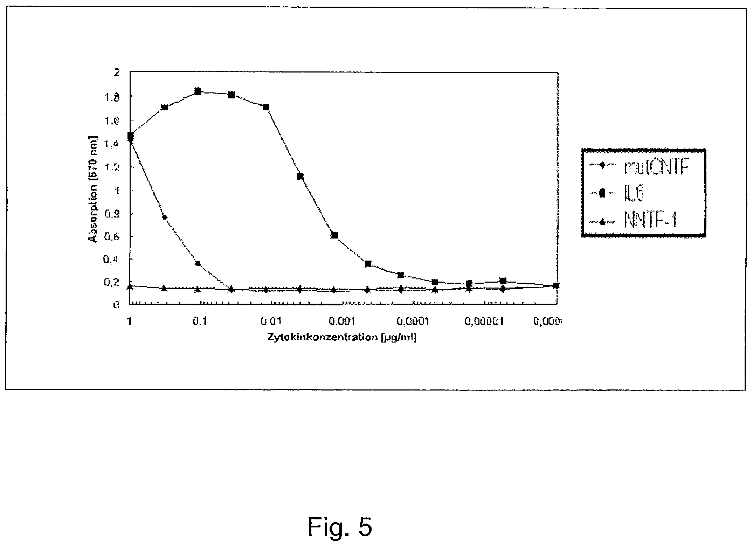

Fig. 4 zeigt die proliferative Antwort von mutCNTF, IL-6 und NNTF-1 auf mit CNTFR, gp130 und LIFR stabil transfizierten BAF/3-Zellen (BAF/3 CNTFR-gp130-LIFR) und Fig. 5 die proliferative Antwort auf mit IL-6Rα, gp130 und LIFR stabil transfizierten BAF/3-Zellen (BAF/3 IL-6R-gp130-LIFR). Beide Zelllinien wurden mit steigender Konzentration von CNTF, IL-6 und NNTF-1 inkubiert und anschließend einem biochemischen Proliferationsassay unterzogen. Als Maß für die Zellteilung wurde photometrisch die Absorption bei 570 nm ermittelt, die direkt proportional zur Anzahl der lebenden Zellen ist.

Die dargestellten Proliferationsassays verdeutlichen, dass mutCNTF und NNTF-1 einerseits dazu in der Lage sind, die Vermehrung der BAF/3 CNTFR-gp130-LIFR-Zellen zu stimulieren, anderseits aber nur mutCNTF und nicht NNTF-1 eine Aktivität auf BAF/3 IL-6R-gp130-LIFR-Zellen besitzt. IL-6 konnte, wie erwartet, nur die Proliferation von BAF/3 IL-6R-gp130-LIFR-Zellen stimulieren (Figs. 4 und 5).

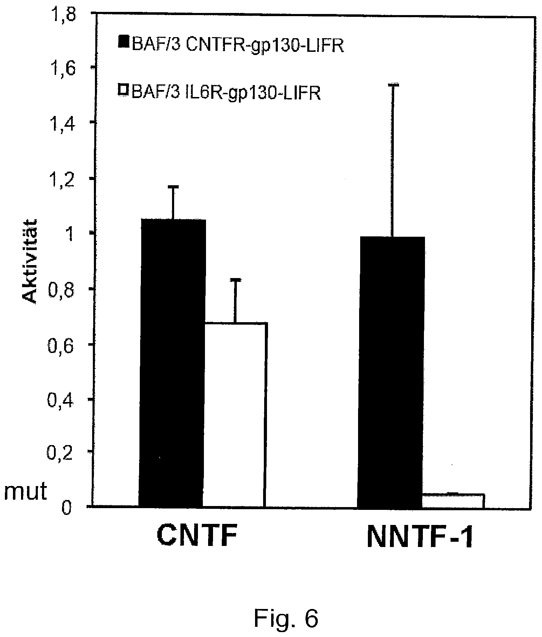

In Fig. 6 ist abschließend NNTF-1 dem ursprünglichen mutCNTF-Molekül funktionell gegenübergestellt. Als repräsentativer Vergleichsparameter diente jeweils die durch mutCNTFinduzierte halbmaximale Proliferationsrate, welche auf BAF/3 CNTFR-gp130-LIFR-Zellen bei einer Zytokinkonzentration von 0,00137 µg/ml und auf BAF/3 IL-6R-gp130-LIFR-Zellen bei 0,333 µg/ml erreicht wurde (s. Figs. 4 und 5). Dabei wird deutlich, dass NNTF-1 im Vergleich zu mutCNTF keine signifikante Aktivität auf BAF/3 IL-6R-gp130-LIFR-Zellen aufweist. Hingegen ist die Aktivität von NNTF-1 auf BAF/3 CNTFR-gp130-LIFR-Zellen vergleichbar mit der von mutCNTF.

Um zu untersuchen, ob mit NNTF-1 der gewünschte Verlust der Interaktion mit dem IL-6R erzielt werden kann, wurde diese erfindungsgemäße Zytokinvariante im Vergleich mit den beiden Zytokinen IL-6 und mutCNTF auf den Zelllinien BAF/3 CNTFR-gp130-LIFR und BAF/3 IL-6R-gp130-LIFR getestet. IL-6 zeigte die bereits bekannte Eigenschaft, BAF/3 IL-6R-gp130-LIFR-Zellen zur Proliferation zu bringen. mutCNTF zeigte diesen Effekt ebenfalls, aber erst bei vergleichbar höheren Konzentrationen. Daraus kann geschlossen werden, dass mutCNTF eine (wenn auch schwache) Affinität zum IL-6R besitzt. NNTF-1 hingegen zeigte auch bei hohen Konzentrationen keinen solchen Effekt: Die Proliferation und das Überleben der BAF/3 IL-6R-gp130-LIFR-Zellen konnten durch NNTF-1 nicht mehr stimuliert werden. Die Versuche mit BAF/3 CNTFR-gp130-LIFR-Zellen zeigten, dass diese, wie erwartet, mit Proliferation auf mutCNTF reagieren. Aus den Ergebnissen lässt sich schlussfolgern, dass NNTF-1, im Gegensatz zu mutCNTF, nicht mehr an den IL-6R binden kann. Dies konnte mittels der Zellproliferation, die nur erfolgt, wenn der Ligand an seinen Rezeptor bindet und darauf hin die intrazelluläre Signalkaskade auslöst, nachgewiesen werden. Zusätzlich wurde aber durch den Aminosäurenaustausch R28E in NNTF-1 die Bindungseigenschaften an den CNTFR nicht messbar beeinflusst. Überraschenderweise reicht der Austausch einer einzigen Aminosäure, um die Affinität von mutCNTF zum IL-6R so zu reduzieren, dass keine Zellproliferation mehr möglich ist. Dadurch wird gleichzeitig eine zentrale Rolle des Arginin-Restes 28 vom humanen CNTF bei der Interaktion zwischen Zytokin und Rezeptor impliziert.

Gegenstand der vorliegenden Erfindung sind somit Nukleinsäuremoleküle, die für CNTF-Varianten codieren, welche anstatt des Arginin-Rests an Position 28 vom humanen Wildtyp-CNTF (SEQ ID:NO 7) einen nicht-positiv-geladenen Aminosäurerest aufweisen.

Gegenstand der Erfindung sind neben den beschriebenen CNTF-Peptiden, die anstatt des Arginin-Rests an Position 28 vom humanen Wildtyp-CNTF (SEQ ID:NO 7) einen nicht-positiv-geladenen Aminosäurerest aufweisen, auch deren biologisch aktive Fragmente. Biologisch aktiv bedeutet, dass die Fragmente gemäß dem in den Beispielen angegebenen Proliferationsassay oder einem anderen dem Fachmann bekannten Nachweisverfahren keine Affinität zum IL-6R aufweisen und demzufolge spezifisch für den CNTFR sind, wie die zugrundeliegenden kompletten Peptide. Bevorzugt handelt es sich um Derivate, bei denen N- oder C-terminal eine oder mehrere Aminosäuren fehlen. Es können jedoch auch Aminosäuren aus der Sequenz deletiert sein. Solche Fragmente weisen bevorzugt nicht mehr als 30 % deletierte Aminosäuren auf.

Gegenstand der Erfindung sind weiterhin solche CNTF-Peptide, bei denen einzelne Aminosäuren ausgetauscht sind. Bevorzugt handelt es sich dabei um konservative Austausche, d. h. Aminosäuren mit ähnlichen Eigenschaften werden ersetzt, beispielsweise Alanin gegen Serin, Leucin gegen Isoleucin, etc. Hier wird bevorzugt, dass nicht mehr als 10 % der Aminosäuren in den Peptiden ersetzt werden.

Darüber hinaus können auch einzelne Aminosäuren durch nicht-natürliche Aminosäuren ersetzt sein, d.h. durch Aminosäuren, die weitere funktionelle Gruppen tragen, beispielsweise Hydroxyprolin, Methylthreonin, Homocystein, etc. Auch in diesem Fall sind bevorzugt nicht mehr als 10 % der Aminosäuren entsprechend modifiziert. Weiterhin können die Peptide Derivatisierungen tragen, beispielsweise amidiert, glycosiliert, acetyliert, sulfatiert oder phosphoryliert sein.

Die vorliegende Erfindung stellt des Weiteren ein Herstellungsverfahren für die erfindungsgemäßen Peptide bereit. Neben der gentechnischen Herstellung der Peptide ist auch die aufbauende Totalsynthese an üblichen Festphasen im Sinne der Merrifield-Synthese oder einer Flüssigphasensynthese möglich. Die Synthesestrategie und der Aufbau der Peptide und von ihnen abgeleiteter Derivate mit den entsprechend geschützten Aminosäuren sind dem Fachmann bekannt.

Die vorliegende Erfindung betrifft außerdem einen Vektor, gekennzeichnet durch ein erfindungsgemäßes Nukleinsäuremolekül sowie eine Wirtszelle, gekennzeichnet durch einen erfindungsgemäßen Vektor, die in der Lage ist, ein erfindungsgemäßes Peptid zu exprimieren.

Wirtszelle, gekennzeichnet durch den Vektor nach Anspruch 5.

Die vorliegende Erfindung betrifft außerdem ein Arzneimittel mit einem erfindungsgemäßen Peptid für verschiedene therapeutische Indikationen. Dazu können die Peptide als hochreine Stoffe oder - wenn für die Verwendung ausreichend - innerhalb eines teilweise gereinigten Peptidgemisches oder als Gemisch mehrerer erfindungsgemäßer Peptide verwendet werden.

Ein weiterer Gegenstand der Erfindung ist ein Arzneimittel mit einem erfindungsgemäßen Peptid zur Behandlung einer mit CNTF im Zusammenhang stehenden Störung oder Erkrankung.

Gegenstand der vorliegenden Erfindung ist somit auch ein Arzneimittel mit einem erfindungsgemäßen Peptid zur Behandlung von Adipositas und damit in Zusammenhang stehenden Erkrankungen wie Adipositas-assoziierter Insulinresistenz, Glucoseintoleranz, Diabetes, Hyperglykämie oder Hyperinsulinämie und/oder zur Verringerung des Körpergewichts.

Gegenstand der vorliegenden Erfindung ist ferner ein Arzneimittel mit einem erfindungsgemäßen Peptid zur Behandlung einer mit CNTF im Zusammenhang stehenden neurologischen oder Differenzierungs-Störung oder -Erkrankung oder einer Nervenschädigung.

Solche mit CNTF im Zusammenhang stehende neurologische oder Differenzierungs-Störungen oder -Erkrankungen oder Nervenschädigungen umfassen degenerative Erkrankungen, wie Netzhautdegenerationen, Erkrankungen oder Störungen, die das Rückenmark, cholinerge Neuronen oder Hippokampus-Neuronen umfassen, oder Erkrankungen oder Störungen, die Motoneuronen umfassen, wie amyotrophe Lateralsklerose oder die des *Nervus facialis,* wie Facialisparese. Andere Erkrankungen oder Störungen, die behandelt werden können, umfassen periphere Neuropathie, Alzheimer-Krankheit, Parkinson-Krankheit, Chorea Huntington oder Muskelatrophie, die beispielsweise von Denervierung, chronischem Missbrauch, Stoffwechselbelastung und Mangelernährung oder einem Zustand wie dem Muskeldystrophiesyndrom, angebotener Myopathie, einer entzündlichen Muskelerkrankung, toxischer Myopathie, Nerventrauma, peripherer Neuropathie, Arzneistoff- oder Toxin-induzierter Schädigung oder Motoneuropathie, resultieren. Die vorliegende Erfindung bezieht sich auch auf Erkrankungen oder Störungen, die aus einer Schädigung des Nervensystems resultieren, wobei eine solche Schädigung durch ein Trauma, einen chirurgischen Eingriff, einen Infarkt, eine Infektion und Malignität oder durch Einwirken eines toxischen Mittels verursacht werden kann.

Die vorliegende Erfindung hat auch als Gegenstand ein Arzneimittel zur Behandlung von Adipositas und damit in Zusammenhang stehenden Erkrankungen und/oder zur Verringerung des Körpergewichts sowie zur Behandlung einer mit CNTF im Zusammenhang stehenden neurologischen oder Differenzierungs-Störung oder -Erkrankung oder Nervenschädigung, das als mindestens einen seiner aktiven Wirkstoffe ein erfindungsgemäßes Peptid enthält und ein geeigneten pharmakologischen Träger umfasst.

Die Erfindung wird anhand der folgenden Beispiele näher beschrieben.

### Beispiel 1: Erzeugung von Punktmutationen durch Splicing by Overlapping Extension (SOE) -PCR

Die SOE-PCR zur Erzeugung einer Punktmutation verläuft in mehreren Schritten. Zusätzlich zu den endständigen Primern einer normalen PCR erstellt man zwei Primer die an exakt komplementären Positionen der Template-DNA liegen und die Position des zu ersetzenden Basentripletts einschließen. Das Template wird somit nicht komplett amplifiziert, sondern in zwei Teilen, jeweils mit dem endständigen 5'-Primer und dem die Mutation enthaltenden 3'-Primer und umgekehrt. Somit entstehen zwei DNA-Fragmente die aufgrund ihrer komplementären Enden in einer finalen Ligations-PCR, welche nur die endständigen Primer verwendet, miteinander verbunden werden können (Fig. 3).

Die Amplifizierung der DNA erfolgte nach folgendem Schema:

| | |
|---|---|
| initiale Denaturierung | 95 °C / 120 s |
| 30 x Denaturierung | 95 °C / 60 s |
| Anlagerung | 55 °C / 60 s |
| Verlängerung | 72 °C / 60 s |
| finale Verlängerung | 72 °C / 300 s |

Die Ligations-PCR wurde unter folgenden Bedingungen durchgeführt:

| | |
|---|---|
| initiale Denaturierung | 95 °C / 60 s |
| 30 x Denaturierung | 95 °C / 60 s |
| Anlagerung | 55 °C / 120 s |
| Verlängerung | 72 °C / 180 s |
| finale Verlängerung | 72 °C / 300 s |

### Primer:

P6forw 5'-CAAGGAAGATTGAGTCAGACCTGACTG-3'
P7rev 5'-CAGTCAGGTCTGACTCAATCTTCCTTG-3'
T7prom 5'-GTAATACGACTCACTATAGGGC-3'
M13rev 5'-CAGGAAACAGCTATGACCAT-3'

### Beispiel 2: Proteinexpression und Reinigung

Für die Proteinexpression der im Expressionsvektor pET-23a(+) (Novagen, Gibbstown, USA) klonierten Inserts NNTF-1 und mutCNTF wurde *E.coli-*Stamm BL21(DE3)pLysS (Novagen, Madison, USA) verwendet. Die Expression erfolgte in einem Liter autoklaviertem und mit entsprechendem Selektionsantibiotikum versetztem LB-Medium. Hierzu wurden in einem ersten Schritt Kolonien der zuvor transformierten Bakterien von der Agarplatte gepickt und in einem kleinen Volumen von 50 ml über Nacht inkubiert, aus diesem anschließend 10 ml in den Expressionsansatz überführt und der Ansatz in einem Inkubator bei 180 rpm und 37 °C bis zu einer OD₆₀₀ von 0,8 geschüttelt. Daraufhin erfolgte die Induktion der Expression mit Hilfe des Lactose-Analogons Isopropyl-β-D-thiogalactopyranosid (IPTG) in einer Endkonzentration von 1 mM. Nach 4 Stunden wurde der Ansatz für 20 min bei 4.000 rpm zentrifugiert und die Pellets bis zu ihrer Reinigung in 50-ml-Zentrifugenröhrchen bei -20 °C gelagert. Der pelletierte Expressionsansatz wurde in einer Serie von 5 Wiederholungen in jeweils 100 ml 1xPBS resuspendiert (initial unter Zusatz von 0,1 % (v/v) Tween^{®} 20), mit Hilfe des Homogenisators SONOPULS HD 2200 (BANDELIN, Berlin, D) 3-mal für eine min auf Eis sonifiziert (30 % Leistung) und erneut 20 min bei 15.000 rpm pelletiert, wobei der Überstand verworfen werden konnte. Nach dem letzten Zentrifugationsschritt erfolgte die Aufnahme der Pellets in insgesamt 12 ml denaturierendem Puffer 1 (6 M GuHCl; 50 mM Tris-HCl pH 8,0). Im Anschluss an die Übernacht-Inkubation bei Zimmertemperatur auf einem Rolltisch wurde die Proteinlösung 10 min bei 10.000 rpm zentrifugiert und der Überstand bis zur weiteren Verarbeitung bei -20 °C gelagert.

Eine weitere Reinigung erfolgte durch Metallchelat-Affinitätschromatographie. Als Trägermaterial der Säule dienten 2 ml Ni-NTA-Agarose (Qiagen, Hilden, D). Diese wurde zweimal mit 10 ml 1x PB S gewaschen, einmal mit 10 ml Puffer 1 äquilibriert und anschließend mit 10 ml Proteinlösung beschickt, woraufhin der Säuleninhalt für 10 Minuten bei Raumtemperatur auf einem Rolltisch inkubierte. Nach dem Durchlauf erfolgten zwei Waschschritte mit jeweils 10 ml Puffer 1 und die Elution des Proteins mit 3 x 4 ml Puffer 2 (6 M GuHCl; 50 mM Tris-HCl pH 8,0; 250 mM Imidazol). Der Erfolg der Reinigung wurde mittels SDS-PAGE überprüft.

Da die in GuHCl gelösten Proteine in denaturierter Form vorlagen erfolgte eine Dialyse, bei der Proteinlösung [1 mg/ml] in einem Volumenverhältnis von 1:1000 zweimal für 12 Stunden gegen 50mM CAPS pH 11,0 bei 4 °C. Die korrekte Faltung wurde mittels CD-Spektroskopie verifiziert.

Zuletzt wurde das FPLC-System ÄKTAexplorer™ (Pharmacia Biotech,Uppsala, S) angewendet, um monomere von aggregierten multimeren Proteinen zu trennen. Nach dem Waschen der an das Explorersystem angeschlossenen HiLoad 16/60 Superdex 75 pg (Säulenvolumen 120 ml) mit 120 ml 0,5 M NaOH erfolgte die Äquilibrierung mit 240 ml entgastem und steril filtriertem 50 mM CAPS pH 11,0 und das Beladen der Säule mit 2 ml der dialysierten Proteinlösung. Während des Trennungsprozesses wurden die Proteine bei 280 nm detektiert, anschließend das fraktionierte Elutionsvolumen durch SDS-PAGE analysiert und mittels Zentrifugationskonzentratoren (Vivaspin 20, MWCO 10.000; Sartorius, Aubagne, F) auf 0,5 bis 1 mg/ml konzentriert.

Die beiden Proteine mutCNTF und NNTF-1 wurden als Monomer dargestellt. IL-6 wurde hergestellt wie beschrieben (Fischer, M, Goldschmitt, J, Peschel, C, Brakenhoff, JP, Kallen, KJ, Wollmer, A, Grotzinger, J and Rose-John, S. I. A bioactive designer cytokine for human hematopoietic progenitor cell expansion. Nat Biotechnol 1997; 15: 142-145).

### Beispiel 3: Aktivitätsbestimmung durch Proliferationsassay

Die Untersuchung der biologischen Aktivität von NNTF-1 und mutCNTF erfolgte auf zwei zuvor stabil mit den benötigten Rezeptorkomponenten transfizierten BAF/3-Zelllininen (48):
Zelllinie: BAF/3 IL-6R-gp130-LIFR
Herkunft: murine pro-B-Zellen, stabil transfiziert mit cDNA für gp130, IL-6R, LIFR
Kultivierung: in Suspension, DMEM + 10 % FCS + 1 % P/S, IL-6 [10 ng/ml]
Zelllinie: BAF/3 CNTFR-gp130-LIFR
Herkunft: murine pro-B-Zellen, stabil transfiziert mit cDNA für gp130, CNTFR, LIFR
Kultivierung: in Suspension, DMEM + 10 % FCS + 1 % P/S, CNTF [10 ng/ml]

Die Zellen wurden dreimal mit sterilem PBS gewaschen, gezählt und in DMEM (+/+) in einer Konzentration von 5 x 10³/100 µl resuspendiert. Nach Zugabe der entsprechenden Zytokine mutCNTF oder NNTF-1 wurden jeweils 100 µl/well in eine 96-well-Platte pipettiert und für zwei Tage anhand einer aufsteigenden Konzentrationsreihe beginnend mit 1 pg/ml und endend mit 1 µg/ml Zytokin kultiviert, um anschließend die Proliferationsassays durchzuführen. Die Zellen wurden bei 37 °C und einem CO₂-Gehalt von 5 % kultiviert. Die Proliferation der Zellen wurde mit Hilfe des CellTiter-Blue Cell Viability Assay (Promega GmbH, Mannheim) nach Protokoll des Herstellers bestimmt und als Maß für die Zellteilung wurde photometrisch die Absorption bei 570 nm ermittelt, die direkt proportional zur Anzahl der lebenden Zellen ist. Alle Werte wurden durch Dreifachbestimmung ermittelt.

## Patentansprüche

1. Nukleinsäuremolekül, ausgewählt aus der Gruppe bestehend aus
a) einem Nukleinsäuremolekül mit der in SEQ ID NO: 1 dargestellten Nukleotidsequenz,
b) einem Nukleinsäuremolekül, das ein Peptid mit der in SEQ ID NO: 2 dargestellten Aminosäuresequenz codiert,
c) einem Nukleinsäuremolekül, dessen Komplementärstrang an ein Nukleinsäuremolekül nach a) oder b) hybridisiert und das für ein Peptid codiert, das an den Ciliary-Neurotrophic-Factor-Rezeptor (CNTFR) bindet, wobei das Peptid mit geringerer Affinität als der Ciliary-Neurotrophic-Factor an den Interleukin-6 Rezeptor (IL-6R) bindet,
d) einem Nukleinsäuremolekül, dessen Nukleotidsequenz von der Nukleotidsequenz eines Nukleinsäuremoleküls nach c) aufgrund des degenerierten genetischen Codes abweicht,
wobei das Codon an Position 82-84 des Nukleinsäuremoleküls nach a) für eine nicht-positiv geladene Aminosäure codiert und das Peptid an der in SEQ ID NO: 2 gezeigten Position 28 einen nicht-positiv geladenen Aminosäurerest aufweist.

2. Nukleinsäuremolekül nach Anspruch 1, **dadurch gekennzeichnet, dass** der nicht-positiv geladene Aminosäurerest ausgewählt ist aus der Gruppe bestehend aus Alanin-, Asparagin-, Asparaginsäure-, Cystein-, Glutamin-, Glutaminsäure-, Glycin-, Isoleucin-, Leucin-, Methionin-, Phenylalanin-, Prolin-, Selenocystein-, Serin-, Threonin-, Tryptophan-, Tyrosin- oder Valin-Rest.

3. Nukleinsäuremolekül nach Anspruch 1 oder 2, **dadurch gekennzeichnet, dass** das Nukleinsäuremolekül ein Peptid codiert, welches an den CNTFR bindet, wobei das Peptid keine oder geringe Affinität zum IL-6R aufweist, und wobei das Peptid eine Aminosäuresequenz aufweist, bei der im Vergleich zu der in SEQ ID NO: 2 dargestellten Aminosäuresequenz einzelne Aminosäuren substituiert sind, wobei nicht mehr als 10% der Aminosäuren ersetzt sind, wobei der nicht-positiv geladene Aminosäurerest in der Position Xaa nicht substituiert ist.

4. Nukleinsäuremolekül mit der in SEQ ID NO: 5 dargestellten Nukleotidsequenz.

5. Vektor, **gekennzeichnet durch** ein Nukleinsäuremolekül mit der Nukleotidsequenz nach einem der vorhergehenden Ansprüche.

6. Wirtszelle, **gekennzeichnet durch** ein Nukleinsäuremolekül nach einem der Ansprüche 1 bis 4.

7. Wirtszelle, **gekennzeichnet durch** den Vektor nach Anspruch 5.

8. Peptid,
(a) das die in SEQ ID NO: 2 oder die in SEQ ID NO: 8 dargestellte Aminosäuresequenz aufweist, wobei Xaa ein nicht-positiv geladener Aminosäurerest ist; oder
(b) bei dem im Vergleich zu einem der Peptide von (a) einzelne Aminosäuren substituiert sind, wobei nicht mehr als 10 % der Aminosäuren ersetzt sind, wobei der nicht-positiv geladene Aminosäurerest in der Position Xaa nicht substituiert ist,
wobei das Peptid an den CNTFR bindet und nicht oder mit geringerer Affinität als der CNTF an den IL-6R bindet.

9. Peptid nach Anspruch 8, **dadurch gekennzeichnet, dass** das Peptid ein zyklisches, amidiertes, acetyliertes, sulfatiertes, phosphoryliertes, glycosiliertes oder oxidiertes Derivat ist.

10. Peptid nach einem der Ansprüche 8 bis 9, **dadurch gekennzeichnet, dass** ≤ 10% der angegebenen Aminosäuren mit nicht-natürlich vorkommenden Aminosäuren substituiert sind.

11. Peptid nach Anspruch 10, **dadurch gekennzeichnet, dass** die nicht-natürlich vorkommenden Aminosäuren ausgewählt sind aus der Gruppe bestehend aus Hydroxyprolin, Methylthreonin oder Homocystein.

12. Arzneimittel mit einem Peptid nach einem der Ansprüche 8-11 zur Verwendung bei der Behandlung einer mit CNTF im Zusammenhang stehenden Störung oder Erkrankung.

13. Arzneimittel zur Verwendung bei der Behandlung nach Anspruch 12, wobei die Erkrankung oder Störung eine Erkrankung oder einer Störung des Nervensystems ist.

14. Arzneimittel zur Verwendung bei der Behandlung nach Anspruch 12, wobei es sich um folgende Erkrankung oder Störung des Nervensystems handelt: degenerative Erkrankungen, wie Netzhautdegenerationen, Erkrankungen oder Störungen, die das Rückenmark, cholinerge Neuronen oder Hippokampus-Neuronen umfassen, oder Erkrankungen oder Störungen, die Motoneuronen umfassen, wie amyotrophe Lateralsklerose oder die des Nervus facialis, wie Facialisparese; periphere Neuropathie, Alzheimer-Krankheit, Parkinson-Krankheit, Chorea Huntington oder Muskelatrophie, die beispielsweise von Denervierung, chronischem Missbrauch, Stoffwechselbelastung und Mangelernährung oder einem Zustand wie dem Muskeldystrophiesyndrom, angeborener Myopathie, einer entzündlichen Muskelerkrankung, toxischer Myopathie, Nerventrauma, peripherer Neuropathie, Arzneistoff- oder Toxin-induzierter Schädigung oder Motoneuropathie, resultieren, sowie Erkrankungen oder Störungen, die aus einer Schädigung des Nervensystems resultieren, wobei eine solche Schädigung durch ein Trauma, einen chirurgischen Eingriff, einen Infarkt, eine Infektion und Malignität oder durch Einwirken eines toxischen Mittels verursacht werden kann.

15. Arzneimittel nach Anspruch 12 zur Verwendung bei der Behandlung nach Anspruch 12, wobei die Erkrankung oder Störung Adipositas oder mit Adipositas assoziierter Insulinresistenz, Glucoseintoleranz, Diabetes, Hyperglykämie oder Hyperinsulinämie ist, und/oder die Behandlung der Verringerung des Körpergewichts dient.

## Claims

1. A nucleic acid molecule, selected from the group consisting of:
(a) a nucleic acid molecule having the nucleotide sequence depicted in SEQ ID NO: 1,
(b) a nucleic acid molecule which encodes a peptide having the amino acid sequence depicted in SEQ ID NO:2,
(c) a nucleic acid molecule, the complementary strand of which hybridizes to a nucleic acid molecule of (a) or (b) and which encodes a peptide that binds to the ciliary neurotrophic factor receptor (CNTFR), wherein the peptide binds to the interleukin-6 receptor (IL-6R) with a lower affinity than the ciliary-neutrophic factor,
(d) nucleic acid molecule, the nucleotide sequence of which differs from the nucleotide sequence of a nucleic acid molecule of (c) due to the degeneration of the genetic code,
wherein the codon in position 82-84 of the nucleic acid molecule of (a) encodes a non-positively charged amino acid and the peptide has a non-positively charged amino residue in position 28 shown in SEQ ID NO: 2.

2. Nucleic acid molecule according to claim 1, wherein the non-positively charged amino acid residue is selected from the group consisting of an alanine, asparagines, aspartic acid, cysteine, glutamine, glutamic acid, glycine, isoleucine, leucine, methionine, phenylalanine, proline, selenocystein, serine, threonine, tryptophane, tyrosine or valine residue.

3. Nucleic acid molecule according to claim 1 or 2, wherein the nucleic acid molecule encodes a peptide which binds to the CNTFR without or with lower affinity towards IL-6R, with an amino acid sequence which differs from the amino acid sequence depicted in SEQ ID NO: 2 by deletion of individual amino acids, wherein not more than 10% of the amino acids are substituted, wherein the non-positively charged amino acid residue is not substituted in position Xaa.

4. Nucleic acid molecule having the nucleotide sequence depicted in SEQ ID NO: 5.

5. Vector, **characterized by** a nucleic acid molecule having the nucleotide sequence according to one of the preceding claims.

6. Host cell, **characterized by** a nucleic acid molecule according to one of claims 1-4.

7. Host cell, **characterized by** the vector according to claim 5.

8. Peptide
(a) having the amino acid sequence depicted in SEQ ID NO: 2 or in SEQ ID NO: 8, wherein Xaa is a non-positively charged amino acid residue, or
(b) wherein not more than 10% of the amino acids are substituted compared with the peptide of (a), wherein the non-positively charged amino acid residue is not substituted in position Xaa, wherein the peptide binds to the CNTFR without or with lower affinity towards IL-6R

9. Peptide according to claim 8, **characterized in that** the peptide is a cyclic, amidated, acetylated, sulphated, phosphorylated, glycosylated or oxidated derivative.

10. Peptide according to any of claim 8 or 9 **characterized in that** ≤ 10% of the recited amino acids are substituted by non-naturally occurring amino acids.

11. Peptide according to claim 10, wherein the non-naturally occurring amino acids are selected from the group consisting of hydroproline, methyl threonine or homocysteine.

12. Medicament with a peptide according to any of claims 8-11 for the use in the treatment of a disorder or disease which is associated with CNTF.

13. Medicament for the use in the treatment according to claim 12, wherein the disease or disorder is a disease or disorder of the nervous system.

14. Medicament for use in the treatment according to claim 12, wherein the disease or disorder of the nervous system is one of the following: degenerative diseases, such as retinal degeneration, diseases or disorders which comprise the spinal chord, cholinergic neurons or hippocampus neurons, or diseases or disorders which comprise motoneurons, such as amyotrophic lateral sclerosis or those of the Nervus facialis, such as facial palsy; peripheral neuropathy, Alzheimer's disease, Parkinson's disease, Huntington's chorea or muscular atrophy, which e.g. result from denervation, chronic abuse, metabolic burden and malnutrition, or from a condition such as muscular dystrophy syndrome, congestive myopathy, an inflammatory muscular disease, toxic myopathy, nerve trauma, peripheral neuropathy, medicament- or toxin-induced damage or motoneuropathy, and diseases or disorders which result from damage of the nervous system, wherein such damage can be caused by a trauma, a surgical intervention, an infarction, an infection, and a malignancy or by exposure to a toxic agent.

15. Medicament according to claim 12 for use in the treatment according to claim 12, wherein the disease or disorder is adipositas or an adipositas-associated insulin resistance, glucose intolerance, diabetes, hyperglycemia or hyperinsulemia and/or the treatment is conducive for the reduction of body weight.

## Revendications

1. Molécule d'acide nucléique, choisie dans l'ensemble formé par les suivantes :
a) une molécule d'acide nucléique comportant la séquence de nucléotides représentée dans la Séquence N° 1,
b) une molécule d'acide nucléique qui code un peptide comportant la séquence d'acides aminés représentée dans la Séquence N° 2,
c) une molécule d'acide nucléique dont le brin complémentaire s'hybride à une molécule d'acide nucléique (a) ou (b) et qui code un peptide qui se lie au récepteur CNTFR (récepteur du facteur neurotrophique ciliaire), lequel peptide se lie au récepteur IL-6R (récepteur de l'interleukine-6) avec une plus faible affinité que le facteur neutrophique ciliaire,
d) et une molécule d'acide nucléique dont la séquence de nucléotides ne diffère de la séquence de nucléotides d'une molécule d'acide nucléique (c) qu'à raison de la dégénérescence du code génétique,
étant entendu que le codon en position 82-84 de la molécule d'acide nucléique (a) code un acide aminé qui ne porte pas de charge positive et que le peptide présente, en la position 28 indiquée dans la Séquence N° 2, un résidu d'un acide aminé non-porteur de charge positive.

2. Molécule d'acide nucléique conforme à la revendication 1, **caractérisée en ce que** le résidu d'acide aminé non-porteur de charge positive est choisi dans l'ensemble constitué par les résidus d'alanine, d'asparagine, d'acide aspartique, de cystéine, de glutamine, d'acide glutamique, de glycocolle, d'isoleucine, de leucine, de méthionine, de phényl-alanine, de proline, de sélénocystéine, de sérine, de thréonine, de tryptophane, de tyrosine et de valine.

3. Molécule d'acide nucléique conforme à la revendication 1 ou 2, **caractérisée en ce que** cette molécule d'acide nucléique code un peptide qui se lie au récepteur CNTFR, lequel peptide n'a pas d'affinité ou n'a qu'une faible affinité pour le récepteur IL-6R, et lequel peptide présente une séquence d'acides aminés dans laquelle, en comparaison de la séquence d'acides aminés représentée dans la Séquence N° 2, des acides aminés individuels sont remplacés, étant entendu qu'il n'y a pas plus de 10 % des acides aminés qui soient remplacés, et que le résidu d'acide aminé non-porteur de charge positive, en la position Xaa, n'est pas remplacé.

4. Molécule d'acide nucléique présentant la séquence de nucléotides représentée en tant que Séquence N° 5.

5. Vecteur **caractérisé par** une molécule d'acide nucléique présentant une séquence de nucléotides conforme à l'une des revendications précédentes.

6. Cellule hôte **caractérisée par** une molécule d'acide nucléique conforme à l'une des revendications 1 à 4.

7. Cellule hôte **caractérisée par** un vecteur conforme à la revendication 5.

8. Peptide
a) qui présente la séquence d'acides aminés représentée dans la Séquence N° 2 ou dans la Séquence N° 8, dans laquelle Xaa représente un résidu d'acide aminé non-porteur de charge positive,
b) ou dans lequel, en comparaison d'un peptide selon (a), des acides aminés individuels sont remplacés, étant entendu qu'il n'y a pas plus de 10 % des acides aminés qui soient remplacés, et que le résidu d'acide aminé non-porteur de charge positive, en la position Xaa, n'est pas remplacé,
lequel peptide se lie au récepteur CNTFR et ne se lie pas au récepteur IL-6R ou s'y lie avec une plus faible affinité que le facteur CNTF.

9. Peptide conforme à la revendication 8, **caractérisé en ce que** le peptide est un dérivé cyclique, amidifié, acétylé, sulfaté, phosphorylé, glycosylé ou oxydé.

10. Peptide conforme à l'une des revendications 8 et 9, **caractérisé en ce qu'**au plus 10 % des acides aminés indiqués sont remplacés par des acides aminés non-naturels.

11. Peptide conforme à la revendication 10, **caractérisé en ce que** les acides aminés non-naturels sont choisis dans l'ensemble formé par l'hydroxy-proline, la méthyl-thréonine et l'homocystéine.

12. Médicament comprenant un peptide conforme à l'une des revendications 8 à 11, pour utilisation dans le traitement d'un trouble ou d'une maladie en relation avec le facteur CNTF.

13. Médicament pour utilisation dans un traitement, conforme à la revendication 12, étant entendu que la maladie ou le trouble est une maladie ou un trouble du système nerveux.

14. Médicament pour utilisation dans un traitement, conforme à la revendication 12, étant entendu qu'il s'agit des maladies ou des troubles suivants du système nerveux : les maladies dégénératives comme les dégénérescences rétiniennes, les maladies ou les troubles qui affectent la moelle épinière, les neurones cholinergiques ou les neurones de l'hippocampe, ou les maladies ou les troubles qui affectent les neurones moteurs, comme la sclérose latérale amyotrophique, ou ceux du nerf facial, comme une parésie faciale ; les neuropathies périphériques, la maladie d'Alzheimer, la maladie de Parkinson, la chorée de Huntington, ou les atrophies musculaires qui résultent par exemple d'une énervation, d'un abus chronique, d'une surcharge métabolique ou d'une dénutrition, ou d'un état comme un syndrome de dystrophie musculaire, une myopathie congénitale, une affection musculaire inflammatoire, une myopathie toxique, un traumatisme nerveux, une neuropathie périphérique, une lésion induite par toxine ou par médicament, ou une neuropathie des nerfs moteurs, ainsi que les maladies ou les troubles qui résultent d'une lésion du système nerveux, étant entendu qu'une telle lésion peut être causée par un traumatisme, une intervention chirurgicale, un infarctus, une infection ou une affection maligne, ou par l'effet d'un agent toxique.

15. Médicament conforme à la revendication 12, pour utilisation dans un traitement, conforme à la revendication 12, étant entendu que la maladie ou le trouble est une obésité, ou une résistance à l'insuline, une intolérance au glucose, un diabète, une hyperglycémie ou une hyperinsulinémie, associé(e) à une obésité, et/ou que le traitement sert à faire diminuer le poids corporel.


### SEQUENCE LISTING

<110> Christian-Albrechts-Universität zu Kiel
<120> Ciliary-Neurotrophic-Factor-Varianten
<130> P 88422
<160> 8
<170> PatentIn version 3.5
<210> 1
   <211> 603
   <212> DNA
   <213> Homo sapiens
<220>
   <221> misc_feature
   <222> (82)..(84)
   <223> n ist a, c, g, oder t, wobei das Codon an der Position 82-84 für eine nicht-positiv-geladene Aminosäure codiert
<400> 1
   atggctttca cagagcattc accgctgacc cctcaccgtc gggacctctg tagccgctct        60
   atctggctag caaggaagat tnnntcagac ctgactgctc ttacggaatc ctatgtgaag       120
   catcagggcc tgaacaagaa catcaacctg gactctgcgg atgggatgcc agtggcaagc       180
   actgatcagt ggagtgagct gaccgaggca gagcgactcc aagagaacct tcaagcttat       240
   cgtaccttcc atgttttgtt ggccaggctc ttagaagacc agcaggtgca ttttacccca       300
   accgaaggtg acttccatca agctatacat acccttcttc tccaagtcgc tgcctttgca       360
   taccagatag aggagttaat gatactcctg gaatacaaga tcccccgcaa tgaggctgat       420
   gggatgccta ttaatgttgg agatggtggt ctctttgaga agaagctgtg gggcctaaag       480
   gtgctgcagg agctttcaca gtggacagta aggtccatcc atgaccttcg tttcatttct       540
   tctcatcaga ctgggatccc agcacgtggg agccattata ttgctaacaa caagaaaatg       600
   tag                                                                     603
<210> 2
   <211> 200
   <212> PRT
   <213> Homo sapiens
<220>
   <221> MISC_FEATURE
   <222> (28) .. (28)
   <223> Xaa steht für jeden nicht-positiv-geladenen Aminosäurerest
<400> 2
   Met Ala Phe Thr Glu His Ser Pro Leu Thr Pro His Arg Arg Asp Leu
   1               5                   10                  15
   Cys Ser Arg Ser Ile Trp Leu Ala Arg Lys Ile Xaa Ser Asp Leu Thr
               20                  25                  30
   Ala Leu Thr Glu Ser Tyr Val Lys His Gln Gly Leu Asn Lys Asn Ile
           35                  40                  45
   Asn Leu Asp Ser Ala Asp Gly Met Pro Val Ala Ser Thr Asp Gln Trp
       50                  55                  60
   Ser Glu Leu Thr Glu Ala Glu Arg Leu Gln Glu Asn Leu Gln Ala Tyr
   65                  70                  75                  80
   Arg Thr Phe His Val Leu Leu Ala Arg Leu Leu Glu Asp Gln Gln Val
                   85                  90                  95
   His Phe Thr Pro Thr Glu Gly Asp Phe His Gln Ala Ile His Thr Leu
               100                 105                 110
   Leu Leu Gln Val Ala Ala Phe Ala Tyr Gln Ile Glu Glu Leu Met Ile
           115                 120                 125
   Leu Leu Glu Tyr Lys Ile Pro Arg Asn Glu Ala Asp Gly Met Pro Ile
       130                 135                 140
   Asn Val Gly Asp Gly Gly Leu Phe Glu Lys Lys Leu Trp Gly Leu Lys
   145                 150                 155                 160
   Val Leu Gln Glu Leu Ser Gln Trp Thr Val Arg Ser Ile His Asp Leu
                   165                 170                 175
   Arg Phe Ile Ser Ser His Gln Thr Gly Ile Pro Ala Arg Gly Ser His
               180                 185                 190
   Tyr Ile Ala Asn Asn Lys Lys Met
           195                 200
<210> 3
   <211> 519
   <212> DNA
   <213> artificial
<220>
   <223> artificial
<400> 3
   atggacctcg cgagccgctc tatctggcta gcaaggaaga ttcgttcaga cctgactgct        60
   cttacggaat cctatgtgaa gcatcagggc ctgaacaaga acatcaacct ggactctgcg       120
   gatgggatgc cagtggcaag cactgatcgt tggagtgagc tgaccgaggc agagcgactc      180
   caagagaacc ttcaagctta tcgtaccttc catgttttgt tggccaggct cttagaagac      240
   cagcaggtgc attttacccc aaccgaaggt gacttccatc aagctataca tacccttctt      300
   ctccaagtcg ctgcctttgc ataccagata gaggagttaa tgatactcct ggaatacaag      360
   atcccccgca atgaggctga tgggatgcct attaatgttg gagatggtgg tctctttgag      420
   aagaagctgt ggggcctaaa ggtgctgcag gagctttcac agtggacagt aaggtccatc      480
   catgaccttc gtttcatttc ttctcatcag actgggtag                             519
<210> 4
   <211> 172
   <212> PRT
   <213> artificial
<220>
   <223> artificial
<400> 4
   Met Asp Leu Ala Ser Arg Ser Ile Trp Leu Ala Arg Lys Ile Arg Ser
   1               5                   10                  15
   Asp Leu Thr Ala Leu Thr Glu Ser Tyr Val Lys His Gln Gly Leu Asn
               20                  25                  30
   Lys Asn Ile Asn Leu Asp Ser Ala Asp Gly Met Pro Val Ala Ser Thr
           35                  40                  45
   Asp Arg Trp Ser Glu Leu Thr Glu Ala Glu Arg Leu Gln Glu Asn Leu
       50                  55                  60
   Gln Ala Tyr Arg Thr Phe His Val Leu Leu Ala Arg Leu Leu Glu Asp
   65                  70                  75                  80
   Gln Gln Val His Phe Thr Pro Thr Glu Gly Asp Phe His Gln Ala Ile
                   85                  90                  95
   His Thr Leu Leu Leu Gln Val Ala Ala Phe Ala Tyr Gln Ile Glu Glu
               100                 105                 110
   Leu Met Ile Leu Leu Glu Tyr Lys Ile Pro Arg Asn Glu Ala Asp Gly
           115                 120                 125
   Met Pro Ile Asn Val Gly Asp Gly Gly Leu Phe Glu Lys Lys Leu Trp
       130                 135                 140
   Gly Leu Lys Val Leu Gln Glu Leu Ser Gln Trp Thr Val Arg Ser Ile
   145                 150                 155                 160
   His Asp Leu Arg Phe Ile Ser Ser His Gln Thr Gly
                   165                 170
<210> 5
   <211> 519
   <212> DNA
   <213> artificial
<220>
   <223> artificial
<400> 5
   atggacctcg cgagccgctc tatctggcta gcaaggaaga ttgagtcaga cctgactgct       60
   cttacggaat cctatgtgaa gcatcagggc ctgaacaaga acatcaacct ggactctgcg      120
   gatgggatgc cagtggcaag cactgatcgt tggagtgagc tgaccgaggc agagcgactc      180
   caagagaacc ttcaagctta tcgtaccttc catgttttgt tggccaggct cttagaagac       240
   cagcaggtgc attttacccc aaccgaaggt gacttccatc aagctataca tacccttctt       300
   ctccaagtcg ctgcctttgc ataccagata gaggagttaa tgatactcct ggaatacaag      360
   atcccccgca atgaggctga tgggatgcct attaatgttg gagatggtgg tctctttgag      420
   aagaagctgt ggggcctaaa ggtgctgcag gagctttcac agtggacagt aaggtccatc      480
   catgaccttc gtttcatttc ttctcatcag actgggtag                             519
<210> 6
   <211> 172
   <212> PRT
   <213> artificial
<220>
   <223> artificial
<400> 6
   Met Asp Leu Ala Ser Arg Ser Ile Trp Leu Ala Arg Lys Ile Glu Ser
   1               5                   10                  15
   Asp Leu Thr Ala Leu Thr Glu Ser Tyr Val Lys His Gln Gly Leu Asn
               20                  25                  30
   Lys Asn Ile Asn Leu Asp Ser Ala Asp Gly Met Pro Val Ala Ser Thr
           35                  40                  45
   Asp Arg Trp Ser Glu Leu Thr Glu Ala Glu Arg Leu Gln Glu Asn Leu
       50                  55                  60
   Gln Ala Tyr Arg Thr Phe His Val Leu Leu Ala Arg Leu Leu Glu Asp
   65                  70                  75                  80
   Gln Gln Val His Phe Thr Pro Thr Glu Gly Asp Phe His Gln Ala Ile
   85 90 95
   His Thr Leu Leu Leu Gln Val Ala Ala Phe Ala Tyr Gln Ile Glu Glu
   100 105 110
   Leu Met Ile Leu Leu Glu Tyr Lys Ile Pro Arg Asn Glu Ala Asp Gly
   115 120 125
   Met Pro Ile Asn Val Gly Asp Gly Gly Leu Phe Glu Lys Lys Leu Trp
   130 135 140
   Gly Leu Lys Val Leu Gln Glu Leu Ser Gln Trp Thr Val Arg Ser Ile
   145 150 155 160
   His Asp Leu Arg Phe Ile Ser Ser His Gln Thr Gly
   165 170
<210> 7
   <211> 200
   <212> PRT
   <213> Homo sapiens
<400> 7
   Met Ala Phe Thr Glu His Ser Pro Leu Thr Pro His Arg Arg Asp Leu
   1 5 10 15
   Cys Ser Arg Ser Ile Trp Leu Ala Arg Lys Ile Arg Ser Asp Leu Thr
   20 25 30
   Ala Leu Thr Arg Ser Tyr Val Lys His Gln Gly Leu Asn Lys Asn Ile
   35 40 45
   Asn Leu Asp Ser Ala Asp Gly Met Pro Val Ala Ser Thr Asp Gln Trp
   50 55 60
   Ser Glu Leu Thr Glu Ala Glu Arg Leu Gln Glu Asn Leu Gln Ala Tyr
   65 70 75 80
   Arg Thr Phe His Val Leu Leu Ala Arg Leu Leu Glu Asp Gln Gln Val
   85 90 95
   His Phe Thr Pro Thr Glu Gly Asp Phe His Gln Ala Ile His Thr Leu
   100 105 110
   Leu Leu Gln Val Ala Ala Phe Ala Tyr Gln Ile Glu Glu Leu Met Ile
   115 120 125
   Leu Leu Glu Tyr Lys Ile Pro Arg Asn Glu Ala Asp Gly Met Pro Ile
       130                 135                 140
   Asn Val Gly Asp Gly Gly Leu Phe Glu Lys Lys Leu Trp Gly Leu Lys
   145                 150                 155                 160
   Val Leu Gln Glu Leu Ser Gln Trp Thr Val Arg Ser Ile His Asp Leu
                   165                 170                 175
   Arg Phe Ile Ser Ser His Gln Thr Gly Ile Pro Ala Arg Gly Ser His
               180                 185                 190
   Tyr Ile Ala Asn Asn Lys Lys Met
           195                 200
<210> 8
   <211> 172
   <212> PRT
   <213> artificial
<220>
   <223> artificial
<220>
   <221> misc_feature
   <222> (15)..(15)
   <223> Xaa steht für jeden nicht-positiv-geladenen Aminosäurerest
<400> 8
   Met Asp Leu Ala Ser Arg Ser Ile Trp Leu Ala Arg Lys Ile Xaa Ser
   1               5                   10                  15
   Asp Leu Thr Ala Leu Thr Glu Ser Tyr Val Lys His Gln Gly Leu Asn
               20                  25                  30
   Lys Asn Ile Asn Leu Asp Ser Ala Asp Gly Met Pro Val Ala Ser Thr
           35                  40                  45
   Asp Arg Trp Ser Glu Leu Thr Glu Ala Glu Arg Leu Gln Glu Asn Leu
       50                  55                  60
   Gln Ala Tyr Arg Thr Phe His Val Leu Leu Ala Arg Leu Leu Glu Asp
   65                  70                  75                  80
   Gln Gln Val His Phe Thr Pro Thr Glu Gly Asp Phe His Gln Ala Ile
                   85                  90                  95
   His Thr Leu Leu Leu Gln Val Ala Ala Phe Ala Tyr Gln Ile Glu Glu
               100                 105                 110
   Leu Met Ile Leu Leu Glu Tyr Lys Ile Pro Arg Asn Glu Ala Asp Gly
           115                 120                 125
   Met Pro Ile Asn Val Gly Asp Gly Gly Leu Phe Glu Lys Lys Leu Trp
       130                 135                 140
   Gly Leu Lys Val Leu Gln Glu Leu Ser Gln Trp Thr Val Arg Ser Ile
   145                 150                 155                 160
   His Asp Leu Arg Phe Ile Ser Ser His Gln Thr Gly
                   165                 170